# EUROPEAN PATENT SPECIFICATION

(11) **EP 2 364 654 B1**
(45) Date of publication and mention of the grant of the patent: **06.03.2013**
(21) Application number: 11250291.9
(22) Date of filing: 11.03.2011
(51) Int. Cl.: A61B 17/34

(54) **Surgical access port**
Chirurgischer Zugriffsanschluss
Port d'accès chirurgical

(30) Priority: 12.03.2010 US 313209 P; 08.02.2011 US 23091
(43) Date of publication of application: 14.09.2011
(73) Proprietor: Covidien LP, Mansfield, MA 02048 (US)
(72) Inventor: Carter, Sally, Wallingford, CT 06492 (US); Hotter, Joseph, 69006 Lyon (FR)
(74) Representative: Soames, Candida Jane

(56) References cited:
- WO-A2-02/34164
- WO-A2-2008/109408

## Description

### CROSS REFERENCE TO RELATED APPLICATION

### BACKGROUND

### Technical field

The present disclosure relates to a surgical access port. More particularly, the present disclosure relates to a surgical access port including a securing member configured for securing the surgical access port relative to tissue.

### Related Art

Surgical access port devices, such as introducers, trocars, cannulas, and so forth are commonly known in the medical art and permit the introduction of a variety of surgical instruments into a body cavity or opening of a patient. In procedures, such as endoscopic or laparoscopic surgeries, an incision is made in tissue for access to an underlying surgical site in the body. These procedures typically employ surgical instruments which are introduced into the body through the port positioned with an opening in tissue. In some instances, the port may be removably secured within the opening in tissue via one or more structures, e.g., a balloon or other suitable structure(s) that is insufflated with a suitable fluid, e.g., saline. In this instance, when the balloon(s) is sufficiently insufflated with the fluid, the balloon engages a body wall or tissue to generally fix the port within the tissue.
WO 02/34164 and WO 2008/109408 disclose a surgical access port in accordance with the preamble of claim 1.

### SUMMARY

Accordingly, in accordance with Claim 1 the invention provides, a surgical access port.

The securing member may be a spring member defining a coiled configuration in at least the initial condition of the securing member. The spring member is at least partially disposed within the lumen of the outer wall of portal member when in the initial condition of the securing member. The spring member may be operatively coupled at one end thereof to the outer wall. The spring member may define an expanded coiled configuration when in the activated condition of the securing member. The recapture instrument may include an elongated member having a spring receiving slot dimensioned to receive a spring segment of the spring member, and may be manipulable whereby the spring receiving slot cooperates with the spring member to return the spring member to the initial condition of the securing member. The recapture instrument may be adapted for rotational movement relative to the outer wall of the portal member whereby with the spring segment within the spring receiving slot of the recapture instrument, relative rotational movement of the recapture instrument will cause the spring member to recoil and return to the initial condition. The distal end of the recapture instrument may be dimensioned for passage through tissue.

In an alternative embodiment, the spring member is mounted about the distal end of the portal member when in the initial condition of the securing member.

### BRIEF DESCRIPTION OF THE DRAWINGS

Embodiments of the present disclosure will be better appreciated by reference to the drawings wherein:

FIG. 1 is a perspective view of a surgical access port apparatus in accordance with the present disclosure;

FIG. 2 is a view of the area of detail represented by the numeral 2 in FIG. 1 illustrating the securing member of the apparatus in an initial condition;

FIG. 3 is an axial view of the apparatus illustrating the securing member in the initial condition;

FIG. 4 is a partial cut-away view of the apparatus of FIG. 1 illustrating a deployment instrument advancing to permit the securing member to assume the activated condition;

FIG. 5 is an axial view similar to the view of FIG. 3 illustrating the securing member in the activated condition;

FIG. 6 is a partially cut-away view of a recapture instrument for use in recoiling and retracting the securing member to return to the initial condition;

FIG. 7 is a view similar to the view of FIG. 6 illustrating the recapture instrument engaging the securing member for return thereof to the initial condition; and

FIG. 8 is a view illustrating an alternate embodiment of the securing member.

### DETAILED DESCRIPTION OF THE EMBODIMENTS

The surgical access port according to the present disclosure is suitable for facilitating the introduction of a surgical instrument into a surgical incision or passage for performing endoscopic or laparoscopic procedures. It is envisioned that the surgical access port may be used in connection with other surgical procedures utilizing natural or formed openings in a body cavity of a patient.

In the drawings and description which follows, the term "proximal" or "leading" refers to the end of the surgical device or instrument of the present disclosure which is closest to the operator, while the term "distal" or "trailing" refers to the end of the device or instrument which is farthest from the operator.

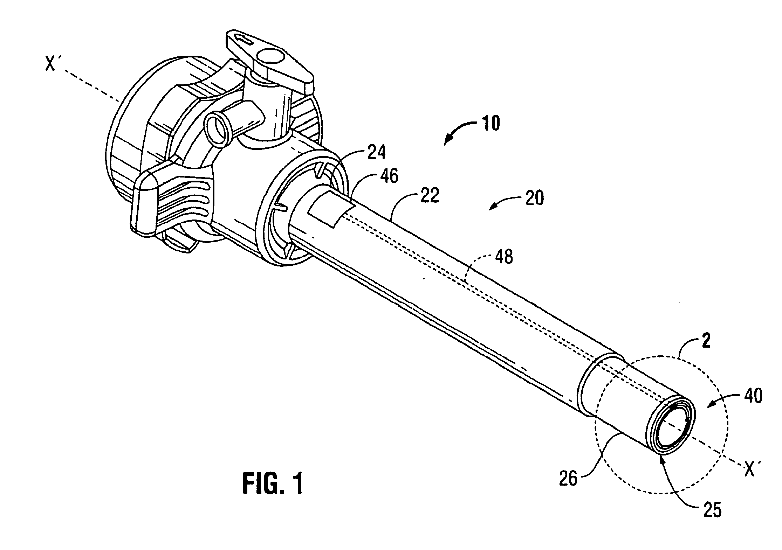

With reference to FIG. 1, a surgical access port apparatus in accordance with the principles of the present disclosure is shown, designated as reference numeral 10. Surgical access port apparatus 10 includes a body portion or portal 20 and securing structure or member 40. Surgical access port apparatus 10 may be any device suitable for the intended purpose of accessing a body cavity, such as a trocar or cannula, and typically defines a passageway permitting introduction of surgical instrumentation therethrough. Instrumentation includes a variety of surgical devices utilized through a portal, such as those used during laparoscopic or endoscopic surgery, as is within the purview of those skilled in the art.

Surgical access port 10 may be used in a variety of surgical applications and is particularly adapted for use in laparoscopic surgery where the peritoneal cavity is insufflated with a suitable gas, e.g., CO₂, to raise the cavity wall from the internal organs therein. Surgical access port 10 includes a securing member 40 having one or more retractable coiled rings or springs that may be deployed to anchor the surgical access port 10 into the surrounding tissue to prevent removal or retropulsion of the portal 20 in the presence of a pressurized body cavity, e.g., an insufflated abdominal cavity.

Portal 20 may be a single monolithically formed unit or composed of several components connected to each other through conventional means, such as, for example, ultrasonic welding, or any other means envisioned by one skilled in the art. Portal 20 may be formed of any suitable medical grade material, including metals such as stainless steel, titanium, and aluminum; other rigid materials, including polymeric materials such as polyetheretherketones, polycarbonate, polypropylene, polyethylene, and composites thereof. Portal 20 may be manufactured for a single use or can be sterilized and reused.

Portal 20 includes outer wall 22 having proximal end 24 and distal end 26. Outer wall 22 defines a longitudinal axis "x" extending along the length of body portion 22 and defines an internal longitudinal passageway or lumen 25 dimensioned to permit passage of surgical instrumentation (not shown). Outer wall 22 includes a generally circular configuration which forms the longitudinal passageway or lumen 25 whereby other surgical instruments may be placed such that body portion 20 aids in the insertion of instruments, implants, and other surgical related apparatus. Further, the shape of outer wall 22 may provide stiffness to outer wall 22 so that it will not bend under the counter force of tissue. Proximal end 24 may include a housing or the like and may incorporate one or more seals for establishing a seal about an inserted object instrument or to close the lumen 25 in the absence of the surgical instrument.

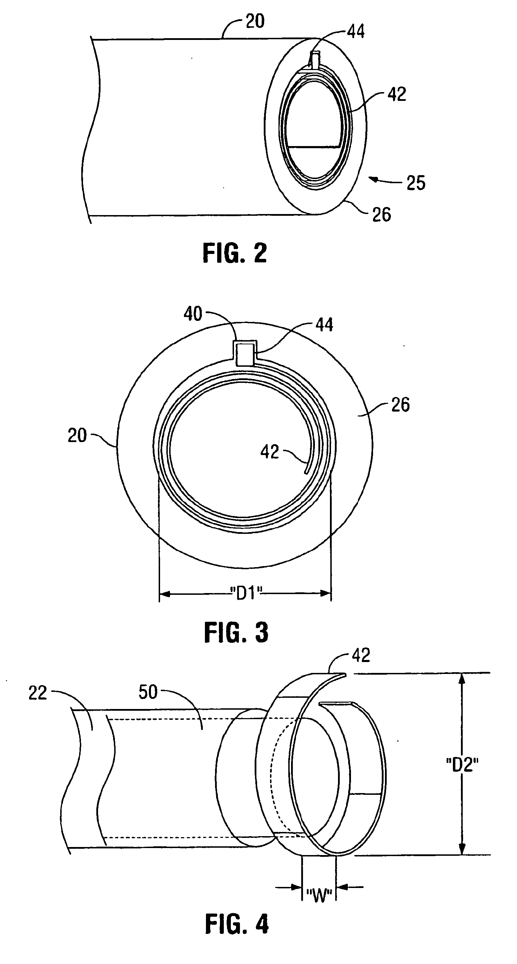

Referring now to FIG. 2, a securing structure in the form of a retractable coil spring 42 is shown operatively disposed on the portal 20. More particularly, spring 42 operatively couples to a distal end 26 of body portion 22. Spring 42 includes a securing end 44 that connects to the distal end 26. More particularly, securing end 44 operably couples to a structure 40 of outer wall 22 having proportional dimensions. In the embodiment illustrated in FIGS. 1-5, the structure 40 is in the form of a slot or slit 40 within outer wall that is configured to couple to the securing end 44 of coil spring 42 via a press fit or friction fit. In embodiments, spring 42 or portion thereof may include other suitable connection structures and or devices (e.g., adhesives, welding, screws or other mechanical fixing mechanisms) configured to secure the spring 42 within the distal end of the portal 20.

Spring 42 is movable from an initial coiled condition to an activated condition. In the activated condition, the spring 42 is configured to securely engage tissue surrounding an opening. In the embodiment illustrated in FIGS. 1-5, spring 42 is shown coiled at a distal end 26 at least partially within lumen 25 of body portion 22. Alternatively, spring 42 may be wrapped (e.g., coiled) around an outer peripheral surface of the body portion 22.

Spring 42 includes dimensions of suitable proportion with respect to the distal end 26 of body portion 22. More particularly, in an initial coiled condition spring 22 includes "n" number of turns and includes a first transverse dimension or outer diameter "D1" that is less than an inner diameter of the body portion 22 and/or at least an inner diameter of the distal end 26 (see FIG. 3, for example). The number of turns "n" that spring 42 includes when the spring 42 is in the initial, coiled condition may depend on a number of variables, such as, for example, the length and/or width of the spring 42, the desired outer diameter "D2" of the spring 42 when the spring 42 is in a subsequent, activated condition, and so forth. The number of turns "n" that spring 42 includes is directly proportional to the amount of force that the spring 42 will exert on the walls of the opening in tissue when the spring 42 is in the uncoiled condition and positioned in the opening in tissue. That is, the greater the amount of turns "n" of the spring 42 the greater the force the spring 42 will exert on the walls of the opening in tissue. Spring 42 includes a second outer dimension or diameter"D2" that is greater than an outer diameter of the distal end 26 of the body 22 when the spring 42 is in the subsequent, uncoiled condition (see FIG. 4, for example). Spring 42 includes a width "w" that is uniform along a length of the spring 42. By increasing the width "w" of the spring 42 the more surface area of the spring 42 is available for gripping tissue, which, in turn, provides a securer and/or tighter fixation of the surgical access port 10 when the spring 42 is in the subsequent, activated and within the opening in tissue. In the alternative, spring 42 may have a circular cross-section.

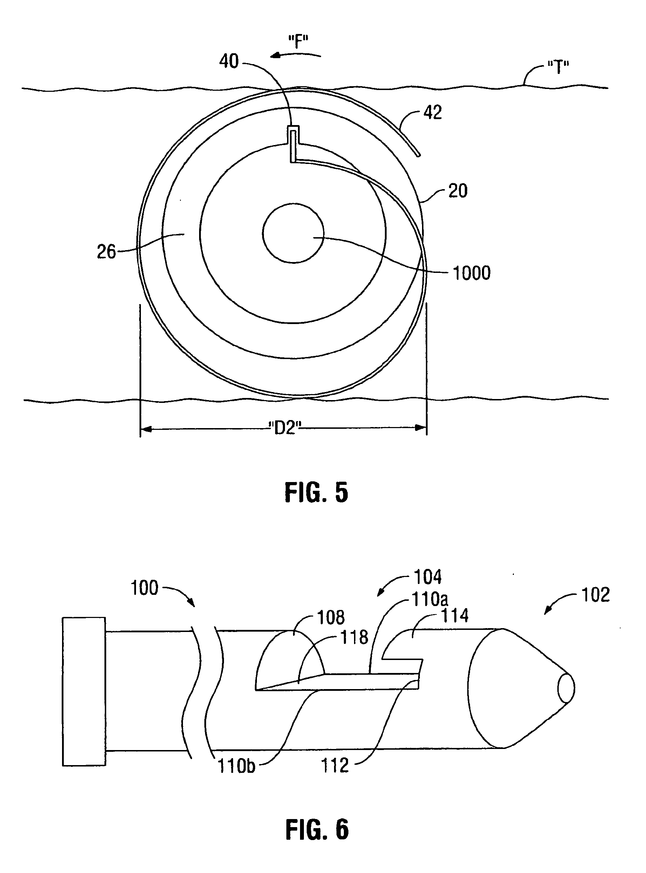

Spring 42 may be formed from any suitable resilient material. For example, in one embodiment, spring 42 is formed from spring steel, shape memory material such as Nitinol or the like. Spring 42 is normally biased to the activated condition of FIGS. 4 and 5 and has sufficient flexibility to be restrained within the lumen 25 of the outer wall 22 in the initial condition of the spring 42. In the activated condition, the coils of spring 42 at least partially unwind to increase the dimension of the spring 42. In embodiments, one or more elements or compounds, such as, for example, carbon or low-alloy steel may added to the spring steel to give it the hardness and yield strength needed in springs so that the steel may return to its original shape after uncoiling, bending, twisting, or other deformation.

An exemplary method of use of surgical access port 10 will now be described. Initially, spring 42 is in the initial coiled condition at least partially within body portion 26 (FIG. 2). Portal 20 may be inserted into an opening, e.g., such as an incision in tissue of a patient, with proximal end 24 available for manipulation by the operator and distal end 26 submerged within the opening in a patient. Once positioned within the opening of a patient, spring 42 is caused to deploy from distal end 26 of portal 20 to grip or otherwise grasp the surrounding walls of the opening in tissue. In embodiments, one or more types of surgical instruments (e.g., an introducer rod 50 depicted in FIG. 4) may be employed to deploy or "push" the spring 42 from the distal end 26 of the portal. Spring 42 in the deployed and actuated condition of FIGS. 4 and 5 will engage surrounding tissue to secure body portion 22 relative to the tissue. Thereafter, a surgical task may be performed within the underlying body cavity with an instrument 1000 introduced through lumen 25 as depicted in FIG. 5. Subsequent to the performance of the surgical procedure, the spring 42 will be returned to its initial condition. In one embodiment, outer wall 22 of portal 20 may be rotated in one direction (e.g., counterclockwise) as shown by directional arrow "F" in FIG. 5. As the outer wall 22 rotates, the spring 42 may be drawn back into the lumen 25 of outer wall 22 and assume the initial condition confined within the outer wall 22.

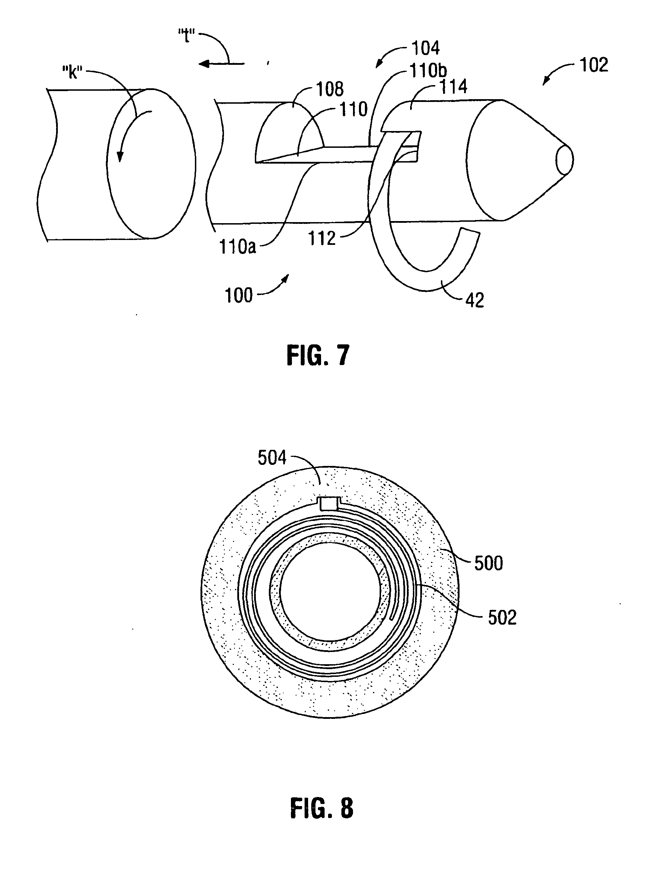

From the foregoing and with reference to the various figure drawings, those skilled in the art will appreciate that certain modifications can also be made to the present disclosure without departing from the scope of the same. For example, surgical access port 10 is typically used with an obturator assembly 100 (FIG. 6) that includes a distal end 102 which may have a blunt, non-bladed end or sharp, bladed end positionable within the passageway of surgical access port 10. The obturator assembly 100 is positioned within the portal apparatus 20 and is utilized to penetrate the abdominal wall. The obturator assembly 100 may then subsequently be removed from surgical access port 10 to permit introduction of surgical instrumentation utilized to perform the procedure through the lumen 25. Accordingly, in embodiments, it may prove useful to provide an obturator assembly 100 and/or distal tip 102 associated therewith that may be utilized to both deploy and/or retract the spring 42. More particularly, the distal tip 102 of the obturator 100 may be configured to recoil and/or retract a securing member associated with the device 10 (see FIG. 6, for example). In this instance, the distal tip 102 may include a generally rounded, circular cross-section that extends along a portion of the distal end 102. The rounded, circular cross-section facilitates deploying the coil spring 42 from the distal end 26 of the body portion 22. That is, the distal tip 102 includes a surface area that is proportioned to force the coil spring 42 distally from the distal end 26 of the body portion 22. A notched or slotted portion 104 is operably disposed along a length of the distal end 102. More particularly, the notched portion 104 may be configured to "hook" and "rotate" the spring 42 such that an operator may recoil and subsequently retract the spring 42 back into the distal end 26 of the body 22. To this end, the notched portion 104 includes a proximal hemispherical sidewall 108. A generally flat surface 110 extends distally from the hemispherical sidewall 108 to a distal sidewall 112 and includes two pronounced side edges 110a and 110b. An overhang 114 extends from the distal sidewall 112 and is defined by an outer peripheral surface of the distal tip 102 of the obturator. The combination of the proximal and distal sidewalls 108 and 112, respectively, side edges 110a and 110b and overhang 112 facilitates in engaging and receiving a segment of spring member 42 as shown in FIG. 7. The obturator 100 may be rotated in, e.g., a counterclockwise direction "k" and simultaneously moved in a proximal direction "t" to recoil and draw the spring 42 within body portion 22 as depicted in FIG. 7.

It is further contemplated that an activation component 46 may be operably connected to an actuating member 48 (shown in phantom in FIG. 1) and configured to deploy and/or retract spring 42 from portal 20. With this purpose in mind, activation component 46 is accessible to an operator at proximal end 24 of portal 20. Activation component 46 and actuating member 48 may be monolithically formed or connected to the portal 20 by means within the purview of those skilled in the art. In embodiments, activation component 46 may be a button, plunger, tab, trigger, or other activation component within the purview of those skilled in the art to help distally and/or proximally translate actuating member 48 with respect to portal 20. Actuating member 48 is mechanical communication with coil spring 42 and is configured to deploy spring 42 for fixation into an opening in tissue. Actuating member 48 may also configured to recoil spring 42 to its initial, coiled condition and, if needed, retract spring 42 back within the body portion 42.

It is contemplated that in embodiments in which portal 20 is used with laparoscopic procedures, surgical access port 10 may also be configured to seal the body opening to maintain the pneumoperitoneum while permitting the introduction of surgical instrumentation. For a more detailed description seals or seal assemblies suitable for use with the surgical access port 10 of the present disclosure reference is made to commonly owned U.S. Patent Nos. 6,702,787 to Racenet et al., 6,482,181, also to Racenet et al. and 6,551,282 to Exline et al.

FIG. 8 illustrates an alternate embodiment of the securing member of the present disclosure. Securing member 500 is substantially similar to the securing member of FIGS. 1-7; however, in accordance with this embodiment, securing member 500 is positioned over cannula member 502 and enclosed within an outer sheath 504. Sheath 504 is retracted relative to cannula member 502 to expose the securing member for deployment and engagement with the tissue. Securing member 500 may be secured to cannula member 502 by any mechanical or adhesive means. An instrument may be advanced within cannula member 502 to perform the desired surgery. Securing member may be retrieved or moved to the initial condition by advancing the outer sheath 504 over the cannula member 502 to thereby constrain the securing member 500 within the confines of the cannula member 502.

It will be understood that various modifications may be made to the embodiments disclosed herein. Therefore, the above description should not be construed as limiting, but merely as an exemplification of preferred embodiments. Those skilled in the art will envision other modifications within the scope of the present disclosure. Such modifications and variations are intended to come within the scope of the following claims.

## Claims

1. A surgical access port (10) apparatus, comprising:
a portal member(20) including an outer wall (22) defining a longitudinal axis and having a proximal end (24), a distal end (26), and a lumen (25) configured to allow a surgical instrument to pass therethrough; and
a securing member (42) operatively connected to the distal end of the outer wall of portal member, the securing member being movable with respect to the portal member between an initial at least partially coiled condition defining a first transverse dimension and an activated condition defining a second transverse dimension greater than the first transverse dimension to engage body tissue to assist in retaining the portal member within the body tissue, **characterised in that** the apparatus further comprises a recapture instrument (100) dimensioned for passage within the lumen of the portal member and being configured and adapted to engage the spring member, the recapture instrument being manipulable to return the spring member to the initial condition of the securing member.

2. A surgical access port apparatus according to claim 1 wherein the securing member is a spring member defining a coiled configuration in at least the initial condition of the securing member.

3. A surgical access port apparatus according to claim 2 wherein the spring member is at least partially disposed within the lumen of the outer wall of portal member when in the initial condition of the securing member.

4. A surgical access port apparatus according to claim 3 wherein the spring member is operatively coupled at one end (44) thereof to the outer wall.

5. A surgical access port apparatus according to claim 3 wherein the spring member defines an expanded coiled configuration when in the activated condition of the securing member.

6. A surgical access port apparatus according to any preceding claim wherein the recapture instrument includes an elongated member having a spring receiving slot, the spring receiving slot dimensioned to receive a spring segment of the spring member, the recapture instrument being manipulable whereby the spring receiving slot cooperates with the spring member to return the spring member to the initial condition of the securing member.

7. A surgical access port according to claim 6 wherein the recapture instrument is adapted for rotational movement relative to the outer wall of the portal member whereby with the spring segment within the spring receiving slot of the recapture instrument, relative rotational movement of the recapturing instrument will cause the spring member to recoil and return to the initial condition.

8. A surgical access port apparatus according to claim 6 wherein the recapture instrument includes a distal end dimensioned for passage through tissue.

9. A surgical access port apparatus according to claim 2 wherein the spring member is mounted about the distal end of the portal member when in the initial condition of the securing member.

## Patentansprüche

1. Chirurgisches Zugriffsanschlussgerät (10), das Folgendes umfasst:
ein Portalelement (20), das eine äußere Wand (22) beinhaltet, die eine Längsachse definiert und ein proximales Ende (24), ein distales Ende (26) und ein Lumen (25) hat, das konfiguriert ist, um zuzulassen, dass ein chirurgisches Instrument durchgeht, und
ein Sicherungselement (42), das operativ am distalen Ende der äußeren Wand des Portalelements angeschlossen ist, wobei das Sicherungselement im Hinblick auf das Portalelement zwischen einem ursprünglichen mindestens teilweise gewendelten Zustand, der eine erste Querdimension definiert, und einem aktivierten Zustand, der eine zweite Querdimension definiert, die größer als die erste Querdimension ist, beweglich ist, um mit Körpergewebe in Eingriff zu kommen, um bei der Aufrechterhaltung des Portalelements innerhalb des Körpergewebes zu helfen, **dadurch gekennzeichnet, dass** das Gerät ferner ein Rückholinstrument (100) umfasst, das zum Durchlass innerhalb des Lumens des Portalelements dimensioniert ist, und konfiguriert und adaptiert ist, um mit dem Federelement in Eingriff zu kommen, wobei das Rückholinstrument manipulierbar ist, um das Federelement zum ursprünglichen Zustand des Sicherungselements zurückzubringen.

2. Chirurgisches Zugriffsanschlussgerät nach Anspruch 1, wobei das Sicherungselement ein Federelement ist, das eine gewendelte Konfiguration in mindestens dem ursprünglichen Zustand des Sicherungselements definiert.

3. Chirurgisches Zugriffsanschlussgerät nach Anspruch 2, wobei das Federelement mindestens teilweise innerhalb des Lumens der äußeren Wand des Portalelements angeordnet ist, wenn es im ursprünglichen Zustand des Sicherungselements ist.

4. Chirurgisches Zugriffsanschlussgerät nach Anspruch 3, wobei das Federelement operativ an einem Ende (44) davon an der äußeren Wand angekoppelt ist.

5. Chirurgisches Zugriffsanschlussgerät nach Anspruch 3, wobei das Federelement eine erweiterte gewendelte Konfiguration definiert, wenn es im aktivierten Zustand des Sicherungselements ist.

6. Chirurgisches Zugriffsanschlussgerät nach einem vorangehenden Anspruch, wobei das Rückholinstrument ein längliches Element beinhaltet, das einen Schlitz zur Federaufnahme hat, wobei der Schlitz zur Federaufnahme dimensioniert ist, um ein Federsegment des Federelements aufzunehmen, wobei das Rückholinstrument manipulierbar ist, wodurch der Schlitz zur Federaufnahme mit dem Federelement kooperiert, um das Federelement zum ursprünglichen Zustand des Sicherungselements zurückzubringen.

7. Chirurgisches Zugriffsanschlussgerät nach Anspruch 6, wobei das Rückholinstrument zur Drehbewegung relativ zur äußeren Wand des Portalelements adaptiert ist, wodurch mit dem Federsegment innerhalb des Schlitzes zur Federaufnahme des Rückholinstruments eine relative Drehbewegung des Rückholinstruments ein Zurückspringen und eine Rückkehr zum ursprünglichen Zustand des Federelements verursacht.

8. Chirurgisches Zugriffsanschlussgerät nach Anspruch 6, wobei das Rückholinstrument ein distales Ende beinhaltet, das zum Durchlass durch Gewebe dimensioniert ist.

9. Chirurgisches Zugriffsanschlussgerät nach Anspruch 2, wobei das Federelement um das distale Ende des Portalelements befestigt ist, wenn es im ursprünglichen Zustand des Sicherungselements ist.

## Revendications

1. Appareil de port d'accès chirurgical (10), comprenant :
un élément de porte (20) comprenant une paroi externe (22) définissant un axe longitudinal et ayant une extrémité proximale (24), une extrémité distale (26) et une lumière (25) configurée pour permettre à un instrument chirurgical de passer à travers ; et
un élément de fixation (42) raccordé en service à l'extrémité distale de la paroi externe de l'élément de porte, l'élément de fixation étant mobile par rapport à l'élément de porte entre un état initial au moins en partie enroulé définissant une première dimension transversale et un état activé définissant une seconde dimension transversale supérieure à la première dimension transversale pour s'engager sur un tissu corporel afin d'aider à retenir l'élément de porte dans le tissu corporel, **caractérisé en ce que** l'appareil comprend en outre un instrument de recapture (100) dimensionné pour un passage à l'intérieur de la lumière de l'élément de porte et configuré et adapté pour s'engager sur l'élément de ressort, l'instrument de recapture étant manipulable pour ramener l'élément de ressort à l'état initial de l'élément de fixation.

2. Appareil de port d'accès chirurgical selon la revendication 1, dans lequel l'élément de fixation est un élément de ressort définissant une configuration enroulée dans au moins l'état initial de l'élément de fixation.

3. Appareil de port d'accès chirurgical selon la revendication 2, dans lequel l'élément de ressort est au moins en partie disposé dans la lumière de la paroi externe de l'élément de porte lorsqu'il se trouve dans l'état initial de l'élément de fixation.

4. Appareil de port d'accès chirurgical selon la revendication 3, dans lequel l'élément de ressort est couplé en service, à son extrémité (44), à la paroi externe.

5. Appareil de port d'accès chirurgical selon la revendication 3, dans lequel l'élément de ressort définit une configuration enroulée en expansion lorsqu'il se trouve à l'état activé de l'élément de fixation.

6. Appareil de port d'accès chirurgical selon l'une quelconque des revendications précédentes, dans lequel l'instrument de recapture comprend un élément allongé ayant une fente réceptrice de ressort, la fente réceptrice de ressort étant dimensionnée pour recevoir un segment de ressort de l'élément de ressort, l'instrument de recapture pouvant être manipulé de sorte que la fente réceptrice de ressort coopère avec l'élément de ressort pour ramener celui-ci à l'état initial de l'élément de fixation.

7. Port d'accès chirurgical selon la revendication 6, dans lequel l'instrument de recapture est à même d'effectuer un mouvement rotatif par rapport à la paroi externe de l'élément de porte, si bien que, avec le segment de ressort qui se trouve dans la fente réceptrice de ressort de l'instrument de recapture, un mouvement rotatif relatif de l'instrument de recapture amènera l'élément de ressort à effectuer une rétraction élastique et à retourner à l'état initial.

8. Appareil de port d'accès chirurgical selon la revendication 6, dans lequel l'instrument de recapture comprend une extrémité distale dimensionnée pour un passage à travers le tissu.

9. Appareil de port d'accès chirurgical selon la revendication 2, dans lequel l'élément de ressort est monté autour de l'extrémité distale de l'élément de porte lorsqu'il se trouve dans l'état initial de l'élément de fixation.
